# EUROPEAN PATENT SPECIFICATION

(11) **EP 3 503 738 B1**
(45) Date of publication and mention of the grant of the patent: **17.03.2021**
(21) Application number: 17781560.2
(22) Date of filing: 25.08.2017
(51) Int. Cl.: A23K 10/30, A23K 50/30, A23K 50/75

(54) **ANIMAL FEED ADDITIVE AND ANIMAL FEED COMPRISING IT**
TIERFUTTERZUSATZSTOFF UND TIERFUTTER, WELCHES DIESER ZUSATZSTOFF ENTHÄLT
ADDITIF D'ALIMENT POUR ANIMAUX ET ALIMENT POUR ANIMAUX LE COMPRENANT

(30) Priority: 26.08.2016 NL 2017376
(43) Date of publication of application: 03.07.2019
(73) Proprietor: Nutreco Nederland B.V., 5831 JN Boxmeer (NL)
(72) Inventor: ROUBOS VAN DEN HIL, Petra Johanna, 5831 JN Boxmeer (NL); BOUWENS, Mark, 5831 JN Boxmeer (NL)
(74) Representative: EP&C
(86) International application number: PCT/NL2017/050560
(87) International publication number: WO 2018/038616

(56) References cited:
- WO-A2-2008/051862
- CN-A- 101 194 674
- CN-A- 101 371 683
- US-A1- 2008 187 574

## Description

### FIELD OF THE INVENTION

The present invention pertains to animal feed in general.

### BACKGROUND OF THE INVENTION

Animal feed is used to support the normal metabolism of animals in order for these animals to stay healthy and grow to their full capabilities. In particular for food producing animals it is very important that the feed optimally supports their health since this is often reflected in their performance as measured by establishing the average daily weight gain of an animal, its weight at the age of slaughter or its age at the slaughter weight. In particular, in growing food producing animals, much attention is given to control the spreading of bacteria within a group of animals kept at a particular production site. In particular since such bacteria might be pathogenic to the animal itself (infection thus reducing the animal's health status and hence its performance) or possibly also to consumers of the animal (humans or other animals). Common methods to reduce spreading of bacteria are to use antibiotics, and/or to vaccinate the animal. Another method used is containment (quarantine) of the animal in combination with sterilizing its feed. This method however is not suitable to grow animals for consumption purposes because of the high costs involved.

US 2008/187574 A1 discloses animal feedstuff having beneficial properties obtained by adding to a substrate one or more fungal species of the kind that excretes substances into said substrate during its growth which are beneficial to the health, growth or weight gain of an animal, or animals to which the feedstuff is intended and allowing the fungus to grow and/or ferment on the substrate. Suitable fungus species include Cordyceps species, Ganoderma species, Grifola species, Trametes Species, Lentinula species, Antrodia species, Agaricus species, Tremella species, Pleurotus species, Lentinus species, Polypore species, Agaricales species, Ascomycetes species and Basidiomycetes species..

CN 101 194 674 A discloses a feed additive which is capable of promoting intestinal function and immunologic function of piglets, wherein Agaricus blazei murrill is used as a strain, a fermentation medium which comprises a carbon source, a nitrogen source and inorganic salt is used to ferment and culture, the obtained products are mixed, frozen and dried according to the weight ratio of the fermentation broth, which is 0.2-5:1. When 0.05-2 percent of the feed additive is added to feed, the feed is capable of reducing the diarrhea rate of the piglets, improving the daily gain, increasing the conversion rate of feed, and increasing the health level and weaning weight of piglets.

WO 2008/051862 A2 discloses compositions for contributing to the health of an animal, e.g., for enhancing the immunocompetence of an animal or human, comprising therapeutically effective amounts of beta-1,3-D-glucan or beta-1,6-D-glucan and an additive selected from the group consisting of vitamin D, zinc, and a combination of vitamin D and zinc.

CN 101 371 683 A discloses an agaricus blazei murill feed additive product and a production method and application thereof. The feed additive is obtained through fungal solid state fermentation and has a strong bacteriostatic effect.

### OBJECT OF THE INVENTION

It is an object of the invention to provide for an animal feed that is particularly suitable to improve animal wellbeing, preferably to reduce or mitigate infection with ubiquitous pathogenic bacteria such as bacteria belonging to the Enterobacteriaceae, in particular Salmonella species.

### SUMMARY OF THE INVENTION

The invention is as set out in the claims. The disclosure pertains to a composition comprising mycelium of Agaricus Blazei Murill and an agent affecting bacteria belonging to Enterobacteriaceae, in particular an agent affecting bacteria belonging to Salmonella and/or Escherichia. The composition of the disclosure generally has a positive effect on the shedding of bacteria of the Enterobacteriaceae, in particular of Salmonella and/or Escherichia. Moreover, the composition is effective against resistant bacteria of the Enterobacteriaceae, in particular of Salmonella and/or Escherichia. The composition may further reduce the bacteria infestation in the infested animal leading to an animal with an improved health status and an increased growth performance. In particular it was found that the colonisation with pathogenic bacteria belonging to the group of the enterobacteriacea is reduced, which is demonstrated by a lower bacteria count in the animal's faeces. With the composition of the invention it may be possible to reduce the amount of preventative antibiotics administered to animals.

The composition of the disclosure include animal feed, a premix of animal feed and a feed additive. Consequently, the disclosure further pertains to a feed additive comprising mycelium of Agaricus Blazei Murill and an agent affecting bacteria belonging to Enterobacteriaceae, in particular an agent affecting bacteria belonging to Salmonella and/or Escherichia. Such a feed additive may comprise further ingredients commonly used in feed additives. The feed additive of the invention may be applied and/or added to a premix of animal feed, to animal feed and/or to drinking water. It may be applied to preserve the premix and/or the feed. The feed additive may further be used to improve the gut health of the animal.

The disclosure further pertains to a premix of animal feed comprising mycelium of Agaricus Blazei Murill and an agent affecting bacteria belonging to Enterobacteriaceae, in particular an agent affecting bacteria belonging to Salmonella and/or Escherichia. The premix of the disclosure may comprise further ingredients commonly used in premixes of animal feed. The premixes of the disclosure generally are further processed and further ingredients are added to form animal feed. Hence, the disclosure also pertains to an animal feed comprising mycelium of Agaricus Blazei Murill and an agent affecting bacteria belonging to Enterobacteriaceae, in particular an agent affecting bacteria belonging to Salmonella and/or Escherichia. The animal feed is generally fed to the animals. *Animal feed* generally comprises animal nutrients such as fats and/or proteins and/or carbohydrates that is fed to an animal to provide in its metabolic requirements. Animal feed can be a nutritionally complete feed (i.e. providing all required nutrients to support a normal metabolism of the animal). Similar ingredients are also contained in a premix of animal feed, which however contains only part of the required nutrients, and need to be mixed with other nutrients or fed separately from these other nutrients.

It is noted that Agaricus Blazei Murill is also called Agaricus Blazei Brasiliensis, Agaricus subrufescens, or Agaricus rufotegulis. In this application, the common name Agaricus Blazei Murill will be used. Herein below, the abbreviation ABM and the term Agaricus Blazei Murill are used interchangeably.

The mycelium of ABM used in the compositions according to the disclosure can be mycelium per se as grown in a liquid matrix or can be mycelium grown on grain. In the present disclosure mycelium of ABM grown on grain is preferred. The grain used for the fermentation can be any grain known in the art suitable for this purpose. Examples of such grains include corn, wheat, millet, sorghum, barley, rye, oat, and soy beans. Preferred grains are millet, oat and rye, and most preferred is rye. More details on mycelium of ABM grown on grain including the preparation techniques are described in WO 2013/171194.

The amount of mycelium of ABM grown on the grain can be chosen as desired where the level of fermentation will determine the amount. Typically, the amount of mycelium is at least 1 weight percent (wt%) and at most 50 wt%, based on the total weight of mycelium and grain. Preferably, the amount of mycelium is at least 5 wt% and most preferably at most 10 wt%, and preferably at most 40 wt%, more preferably at most 35 wt% and most preferably at most 30 wt%, based on total weight of mycelium and grain. Methods to determine the amount of mycelium, such as measuring the ergosterol content, are described in WO 2013/171194.

In one aspect, the amount of mycelium of ABM is at least 0.001 wt%, preferably at least 0.01 wt%, more preferably at least 0.1 wt% and most preferably at least 0.5 wt%, and at most 30 wt%, preferably at most 20 wt%, more preferably at most 15 wt%, and most preferably at most 10 wt%, based on the total weight of the composition.

In another aspect, the amount of mycelium of ABM is at least 0.1 wt%, preferably at least 0.5 wt%, more preferably at least 1 wt% and most preferably at least 2 wt%, and at most 30 wt%, preferably at most 20 wt%, more preferably at most 15 wt%, and most preferably at most 10 wt%, based on the total weight of the feed additive.

In yet another aspect, the amount of mycelium of ABM is at least 0.001 wt%, preferably at least 0.01 wt%, more preferably at least 0.1 wt% and most preferably at least 0.15 wt%, and at most 5 wt%, preferably at most 2 wt%, more preferably at most 1 wt%, and most preferably at most 0.5 wt%, based on the total weight of the animal feed.

In yet a further aspect, the amount of mycelium of ABM is at least 0.01 wt%, preferably at least 0.1 wt%, more preferably at least 1 wt% and most preferably at least 1.5 wt%, and at most 15 wt%, preferably at most 12 wt%, more preferably at most 10 wt%, and most preferably at most 5 wt%, based on the total weight of the premix of animal feed.

The agent affecting bacteria belonging to enterobacteriaceae of the disclosure can be any such agent known in the art. Such agents include pharmaceutically active ingredients and nutritionally active ingredients. Pharmaceutically active ingredients are agents that are capable of killing the bacteria, and include antibiotics such as cephalosporins and fluoroquinolones. The combination with the mycelium of the invention may allow for a reduction in the amount of pharmaceutically active ingredient administered to the animal preventatively or curatively. Nutritionally active ingredients are agents that affect the gut health and/or the immune system of the animal and/or are capable of reducing the growth of the bacteria either in the animal feed and/or in the animal's gut. Examples of such agents include organic acids such as formic acid, lactic acid, butyrate and benzoic acid; prebiotic carbohydrates such as mono-, di- and polysaccharides of mannans, mono-, di- and polysaccharides of arabinoxylans, mono-, di- and polysaccharides of inulin-type fructans, galacto-oligosaccharides, xylo-oligosaccharides and chyto-oligosaccharides; prebiotic fibres such as pea fibre, palm kernel fibre, coconut fibre, wheat fibre, oat fibre and further processed products; plant extracts such as essential oils, carvacrol, thymol, eugenol, cinnemaldehyde and capsacin; and probiotics or microbial-derived products such as Bacillus spp., Saccharomyces spp., Lactobacillus spp. And fermented products or co-products thereof such as yeast cell-wall derived products Preferred agents are selected from the group consisting of formic acid, lactic acid, benzoic acid, hydrolysed copra meal, yeast cell wall derived products, manno-oligosaccharides and β-1,4-mannobiose. Even more preferred is hydrolysed copra meal, yeast cell wall derived products, manno-oligosaccharides and β-1,4-mannobiose. Even more preferred are manno-oligosaccharides and β-1,4-mannobiose. Most preferred is β-1,4-mannobiose.

In one aspect, the amount of agent affecting bacteria belonging to enterobacteriaceae is at least 0.001 wt%, preferably at least 0.01 wt%, more preferably at least 0.1 wt% and most preferably at least 0.5 wt%, and at most 30 wt%, preferably at most 20 wt%, more preferably at most 15 wt%, and most preferably at most 10 wt%, based on the total weight of the composition.

In another aspect, the amount of agent affecting bacteria belonging to enterobacteriaceae is at least 0.1 wt%, preferably at least 0.5 wt%, more preferably at least 1 wt% and most preferably at least 2 wt%, and at most 30 wt%, preferably at most 20 wt%, more preferably at most 15 wt%, and most preferably at most 10 wt%, based on the total weight of the feed additive.

In yet another aspect, the amount of agent affecting bacteria belonging to enterobacteriaceae is at least 0.001 wt%, preferably at least 0.01 wt%, more preferably at least 0.1 wt% and most preferably at least 0.15 wt%, and at most 5 wt%, preferably at most 2 wt%, more preferably at most 1 wt%, and most preferably at most 0.5 wt%, based on the total weight of the animal feed.

In yet a further aspect, the amount of agent affecting bacteria belonging to enterobacteriaceae is at least 0.01 wt%, preferably at least 0.1 wt%, more preferably at least 1 wt% and most preferably at least 1.5 wt%, and at most 15 wt%, preferably at most 12 wt%, more preferably at most 10 wt%, and most preferably at most 5 wt%, based on the total weight of the premix of animal feed.

In one embodiment of the invention, the weight ratio of mycelium of ABM and the agent affecting bacteria belonging to enterobacteriaceae in the compositions of the invention is at least 0.01, preferably at least 0.1, more preferably at least 0.3 and most preferably at least 0.5, and at most 50, preferably at most 20, more preferably at most 10 and most preferably at most 5.

The compositions of the disclosure are effective against bacteria belonging to enterobacteriaceae. In particular, the compositions of the invention are effective against enterobacteriaceae that are pathogens for animals in particular for livestock such as chickens, pigs and ruminants. Such bacteria include Salmonella such as S. Typhimurium, S. Infantis, S. Heidelberg, S. Enteritidis, S. Tennessee, S. Brandenburg, S. Newport, S. Mbandaka, S. Cubana, S. Senftenberg, S. Yoruba, S. Derby, S. Kedoudgo, S.Cholerae-suis, S. Livingstone, S. Lexington, S. Gallinarum and S. Agona, and Escherichia such as E. Coli and E. Vulnerus. Preferred are agents affecting one or more bacteria selected from the group of S. Typhimurium, S. Enteritidis and E.Coli.

It is noted that the mycelium of ABM and the agent affecting bacteria belonging to enterobacteriaceae do not need to be present in the same feed material at the same time. It is essential that the various feed components (solid feed, drinking water etc.) taken by an animal as a whole comprise both ingredients, such that at least in the gastro-intestinal tract both ingredients are combined and act in accordance with the present invention.

The invention also pertains to a kit-of-parts comprising a first constituting part that comprises Agaricus Blazei Murril (ABM) mycelium and a second constituting part comprising one or more agent affecting bacteria belonging to enterobacteriaceae, and optionally an instruction to orally administer both these parts of the kit to an animal. It is noted that for the sense of the present invention the parts do not need to be present in one single container. It is foreseen that the parts are provided in separate containers, not packed together, but with the clear intention (for example by indications provided on a web-site, separate leaflet, etc.) to be used according to the teaching of the present invention, for example by adding one or both parts to animal feed, and/or one or both parts to the drinking water that is offered to the animal in conjunction with its feed.

The invention also enables a method to feed an animal by providing feed to the animal comprising Agaricus Blazei Murril mycelium and one or more agent affecting bacteria belonging to enterobacteriaceae. This can be accomplished for example by having both ingredients present in the animal feed, or by feeding the animal with a first substance comprising the mycelium of ABM and a second substance comprising the agent affecting bacteria belonging to enterobacteriaceae (for example drinking water in which the acids are present). The invention also enables a method to provide animal feed by mixing Agaricus Blazei Murril mycelium and one or more agent affecting bacteria belonging to enterobacteriaceae with protein and/or carbohydrates and/or fats to provide the feed.

In a first embodiment of the feed according to the invention, the ABM mycelium is present in the animal feed in an amount of at least 0.01 kg per ton of total daily intake, preferably at least 0.02, more preferably at least 0.05 kg per ton, and at most 10 kg per ton of total daily intake of feed, preferably at most 5 kg per ton, more preferably at most 2 kg per ton, and most preferably at most 1 kg per ton of total daily intake. In the context of this application, the *total daily intake of feed* is the complete mass of feed an animal takes per day, excluding drinking water. This amount can be present in a nutritional complete feed as such, at a level of 0.01 to 5 kg per ton of that feed material, or may for example be present in a concentrated feed material (exceeding 5 kg/ton feed material) as long as the amount per total daily intake of feed is between 0.01 and 5 kg ABM mycelium per ton. In particular, the ABM mycelium is fed at an amount of 0.05 to 2 kg per ton of total daily intake of feed. These amounts appear to suffice for use according to the current invention.

In a further embodiment the one or more agent affecting bacteria belonging to enterobacteriaceae are present in present in the animal feed in an amount of at least 0.01 kg per ton of total daily intake, preferably at least 0.02, more preferably at least 0.05 kg per ton, and at most 10 kg per ton of total daily intake of feed, preferably at most 5 kg per ton, more preferably at most 2 kg per ton, and most preferably at most 1 kg per ton of total daily intake.

The disclosure further pertains to the use of the composition of the invention against resistant bacteria of the Enterobacteriaceae, in particular of Salmonella and/or Escherichia. With the term "resistant bacteria" is meant bacteria that are resistant to conventional antibiotics. Examples of such resistant bacteria include cefotaxime-resistant Escherichia Coli, carbapenem-resistant enterobacteriaceae and extended spectrum beta lactamase-producing Escherichia coli (ESBL-producing E. coli). Preferably, the disclosurepertains to the use of the inventive composition in animal feed against resistant bacteria of the Enterobacteriaceae, in particular of Salmonella and/or Escherichia.

### EXAMPLES

Example 1 describes an in vitro model study for assessing the effect of an antimicrobial on bacterial growth.
Example 2 describes an in vivo study for assessing the effect of ABM mycelium combined with organic acids on bacterial shedding.
Example 3 describes a second in vivo study for assessing the effect of ABM mycelium combined with organic acids on bacterial shedding.
Example 4 describes an in vitro study on bacterial growth with different treatments
Example 5 describes an in vivo study for assessing the effect of ABM mycelium combined with organic acids on bacterial shedding
Example 6 describes an in vivo study with broilers assessing the transmission of Salmonella

Figure 1 shows the effect of ABM mycelium combined with organic acids on the shedding of Salmonella.
Figure 2 shows the effect of ABM mycelium combined with organic acids on diarrhea.
Figure 3 shows the effect of ABM mycelium combined with organic acids on the feed intake.
Figure 4 shows the effect of ABM mycelium combined with organic acids on the feed efficacy
Figure 5 shows the effect of ABM mycelium combined with organic acids on the shedding of enterobacteriaceae in further in vivo studies.

### Example 1

Example 1 describes an in vitro model study for assessing the effect of ABM mycelium on bacterial adhesion. In this method the adhesion of *Salmonella typhimurium* to ABM mycelium is assessed.

Use was made of a 96 wells plate on which the ABM mycelium was coated. For this, the ABM mycelium (in this and each case below a fermented rye product was actually used, in which product the amount of ABM mycelium was about 15% w/w) was suspended in PBS to a final concentration of 1% (w/v) and mixed thoroughly. Subsequently the suspension was centrifuged to remove insoluble material. Thereafter, the supernatant was used for coating the wells of the microtiter plate. For the adhesion assessment, a *Salmonella typhimurium* suspension was added to the microtiter plate. The plate was then incubated for 30 minutes and after this incubation step washed with PBS. Subsequently growth medium was added to the wells and the time to onset OD600 value was determined. The optical density (OD) measurement was used as a tool to compare numbers of adhered bacteria to the coated wells of the 96 wells plate with different compounds. The initial cell density of adhered bacteria correlates with the time-dependent detection of the growth by optical density measurement. A shorter time to onset OD600 value represents more adhesion of bacteria to the substrate, and hence an expected higher decrease of in vivo growth.

The results for the test with *Salmonella typhimurium* showed that the average time to onset OD600 was 4.9 hours (± 0.3h) as compared to the control (only PBS) which had an average time to onset OD600 of 7.3 hours (± 0.1h). About twenty other compounds which were suspected of having a potential effect an adhesion (compounds not indicated in this example) showed an average time to onset OD600 generally between 5 and 8.5 hours.

In a second in vitro study the test was repeated, and additionally the effect on *Salmonella enteritidis* and *E. coli was* measured. Also, the amount of ABM mycelium was used in the full amount (see above; denoted "100%"), half of this amount ("50%") and a quarter of this amount ("25%)". The results are indicated here beneath in Table 1.

**Table 1. Effect of ABM mycelium in various amounts on the adhesion of various enterobacteriaceae, by measuring the time to onset OD600 in hours.**

| Compound | *S. thyphimurium* | *S. enteritidis* | *E. coli* |
|---|---|---|---|
| Control | 7.0 | 6.3 | 7.1 |
| ABM 100% | 6.0 | 5.5 | 5.7 |
| ABM 50% | 5.7 | 5.5 | 5.9 |
| ABM 25% | 5.7 | 5.5 | 6.4 |

From the model studies it appears that mycelium of ABM has a significant effect on the adhesion of various enterobacteriaceae. The effect appears to be independent of the type of bacterium despite the fact that in particular the Escherichia bacteria are of a completely different species than the Salmonella bacteria. The amount of ABM mycelium does not appear to be critical to obtain the adhesion effect as such.

### Example 2 (reference example)

Example 2 describes an in vivo study for assessing the effect of ABM mycelium combined with organic acids on bacterial shedding. In this study it was assessed whether the effect seen in vitro (see Example 1) indeed corresponds to in vivo bacterial shedding. In particular, it was assessed whether by introducing ABM mycelium in the feed of the pigs, in this case combined with an organic acids blend, the shedding of viable bacteria could be reduced. As controls, a negative control using the regular feed was used, and as a positive control the same feed with added butyrate, a particular short chain fatty acid that is commercially used in poultry feed to reduce bacterial shedding. The organic acid blend was a regular C₁-C₁₆ organic acid blend containing a combination of formic and lactic acid, added at 4 litres per 100 kg.

A total of 24 Topi*Hypor boar piglets were used. Only healthy male animals which did not receive antibiotics and which were negative for Salmonella (determined by qualitative examination of the faeces) were included in the study. Animals were identified by uniquely numbered ear tags. Animals were divided over three treatment groups (8 animals per group) by weight and litter.

Piglets were individually housed (0.8x1.6m) directly after weaning (24 days of age+/- 3 days) in pens containing tenderfoot slatted floors. The first 24 hours after weaning continuous light was provided, thereafter 16 hours light and 8 hours darkness. Piglets received feed and drinking water ad lib. The different treatments were administered in the feed during the total study period (from weaning until the end of the study) as indicated below in Table 2.

**Table 2 Feed treatments**

| Treatment | No. of animals | Additives | Inclusion level |
|---|---|---|---|
| Negative control | 8 | - | - |
| Butyrate | 8 | Butyrate + acid blend | 6 kg/ton + 4L/ton |
| ABM mycelium | 8 | ABM + acid blend | 2 kg/ton + 4L/ton |

After 10 days piglets were orally infected with *Salmonella typhimurium* (in BHI medium) given by a pre-inoculated feed matrix containing 1 ml 1*10⁹ cfu/ml. Oral infection was performed in this way during 7 consecutive days.

Faecal sampling was performed at day 1, 2, 3, 4, and 7 post Salmonella infection. Samples were stored at 4 degrees and analyzed the next day. Samples were diluted and homogenized in BPW containing novobiocin. Serial dilutions were made and plated onto selective chromogenic agar plates, and incubated o/n at 37°C. Typical *Salmonella* colonies were counted and the amount (cfu/gram) was calculated. Of each sample two presumptive *Salmonella* colonies were confirmed by qPCR for both *Salmonella* and *Salmonella typhimurium.* When no colonies were observed in the lowest dilution plates the samples were screened for Salmonella presence (qualitative) after pre-enrichment by the conventional MSRV/XLD method.

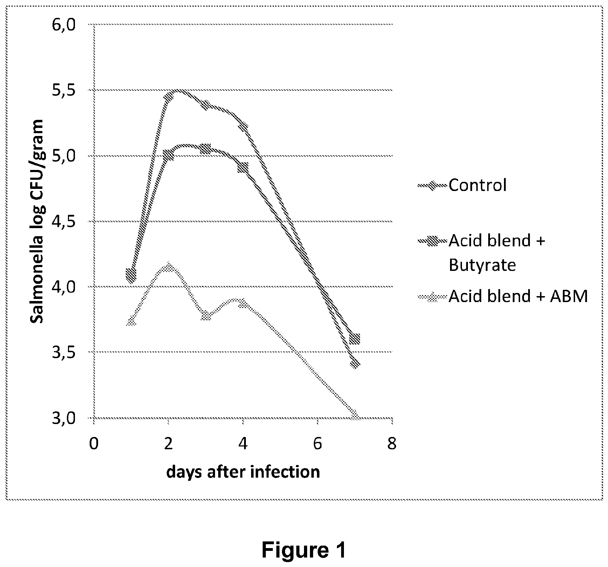

The results are indicated in Figure 1, which shows the effect of ABM mycelium combined with organic acids on the shedding of Salmonella. It appears that the combination of mycelium of ABM and organic acids indeed has a significant effect on the shedding of viable salmonella bacteria. In particular, the effect is very large when compared to butyrate, a compound that is used in poultry for this purpose. It is thus also clear that the in vitro model (Example 1) is predictive for the in vivo reduction of bacterial shedding.

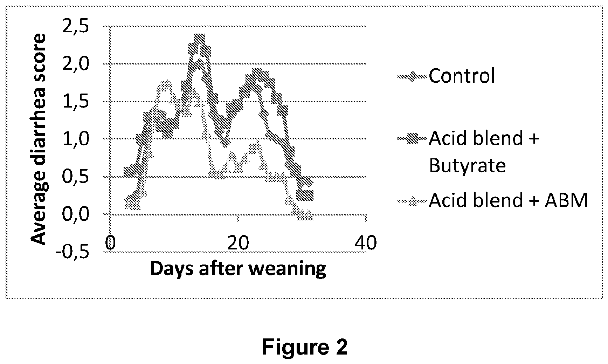

Figure 2 shows the effect on diarrhoea. A faeces scoring was performed daily from day 3 after weaning until the end of the study. Diarrhea score was determined as: 0 = normal faeces; 1 = flat faeces; 2 = wet faeces; 3 = watery faeces. The results as depicted in Figure 2 show a significant reduction of the ABM mycelium on diarrhoea.

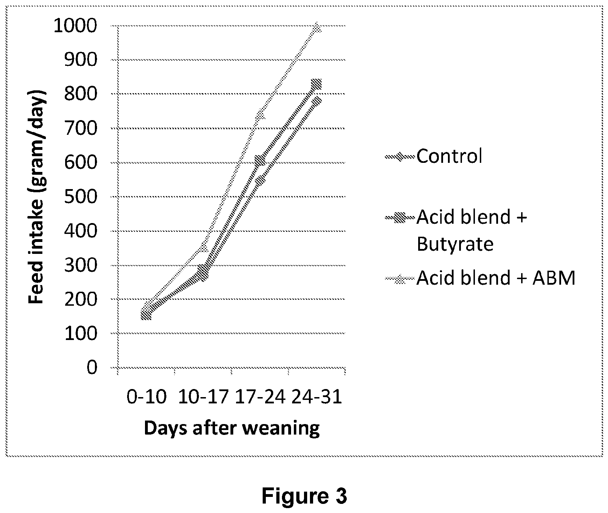

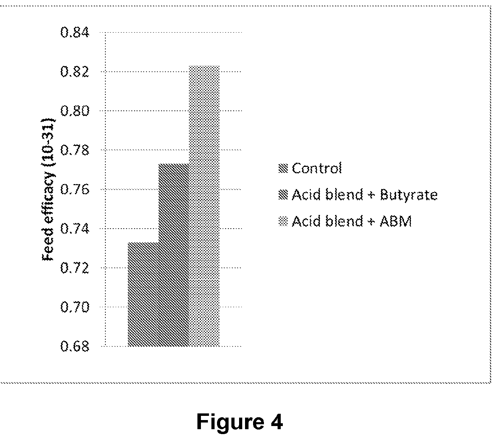

To assess performance, piglets were inspected daily. Body weight and feed intake were determined at weaning, before infection, and 7, 14, and 21 days after infection (day 0, 10, 17, 24, and 31). Feed efficacy was determined as gram growth/gram feed intake. Figure 3 shows the effect of ABM mycelium combined with organic acids on the feed intake. Figure 4 shows the effect of ABM mycelium combined with organic acids on the feed efficacy. The results show a significant positive impact on performance due to the presence of ABM mycelium in the feed.

### Example 3 (reference example)

Example 3 describes a second in vivo study for assessing the effect of ABM mycelium combined with organic acids on bacterial shedding. In this study, as a positive control the acid blend on itself was used (thus without the ABM mycelium).

A total of 36 Topi*Hypor boar piglets were used. Only healthy male animals which did not receive antibiotics and which were negative for Salmonella (determined by qualitative examination of the faeces) were included in the study. Animals were identified by uniquely numbered ear tags. Animals were divided over three groups (12 animals per group) by weight and litter.

Piglets were individually housed (0.8x0.8m) directly after weaning (24 days of age+/- 3 days) in pens containing tenderfoot slatted floors. The first 24 hours after weaning continuous light was provided, thereafter 16 hours light and 8 hours darkness. Piglets received feed and drinking water ad lib. The different treatments were administered in the feed during the total study period (from weaning until the end of the study) as indicated below in Table 3.

**Table 3 Feed treatments**

| Treatment | No. of animals | Acid blend | ABM |
|---|---|---|---|
| Negative control | 12 | None | - |
| Acid blend | 12 | 4 L/ton | - |
| Acid blend + ABM mycelium | 12 | 4 L/ton | 2 kg/ton |

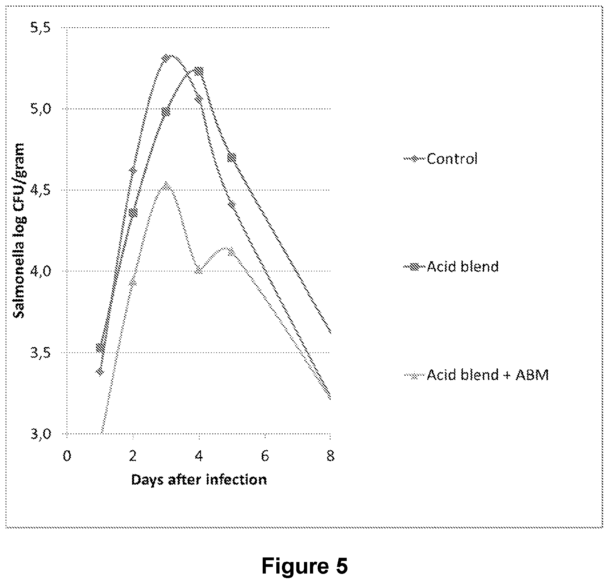

After 8 days piglets were orally infected with *Salmonella typhimurium* (in BHI medium) given by a pre-inoculated feed matrix containing 1 ml 1*10⁹ cfu/ml. Oral infection was performed in this way during 7 consecutive days.
Faecal sampling was performed at day 1, 2, 3, 4, and 5 post Salmonella infection. Samples were stored at 4 degrees and analyzed the next day. Samples were diluted and homogenized in BPW containing novobiocin. Serial dilutions were made and plated onto selective chromogenic agar plates, and incubated o/n at 37°C. Typical *Salmonella* colonies were counted and the amount (cfu/gram) was calculated. Of each sample two presumptive *Salmonella* colonies were confirmed by qPCR for both *Salmonella* and *Salmonella typhimurium.* When no colonies were observed in the lowest dilution plates the samples were screened for Salmonella presence (qualitative) after pre-enrichment by the conventional MSRV/XLD method. The results are indicated in Figure 5 and correspond to the results as indicated in Figure 1.

The above in vivo experiment was repeated to assess the effect on Escherichia coli shedding by pigs. The experiment was run in correspondence with the salmonella experiment as described here above, with 10 animals being used per group. The results showed that on the day of artificial E. coli infection, none of the animals were positive in their faeces for E. coli. At day 12, over 70% of the animals were positive in each group. Two days later, in the two control groups (negative control and acid blend group) the percentage of positive animals was 60%, whereas in the ABM group no shedders (0% of the animals tested positive for E. coli) were present at all.

### Example 4

An in vitro model study was conducted according to the protocol described in Example 1. In this method the adhesion of Salmonella typhimurium and of Escherichia Coli to ABM mycelium on rye, to β-1,4-mannobiose, and to three combinations of ABM mycelium on rye and β-1,4-mannobiose with different combinations, i.e. 75:25, 50:50 and 25:75. The total amount of the agents is the same in all experiments.

The results for the test with *Salmonella typhimurium* are shown in the Table below.

**Table 4. Effect of ABM mycelium on rye and β-1,4-mannobiose in various amounts on the adhesion of various enterobacteriaceae, by measuring the time to onset OD600 in hours.**

| Compound | *S. thyphimurium* | *E. coli* |
|---|---|---|
| Control | 6.95 | 7.10 |
| ABM 100%: β-1,4-mannobiose 0% | 5.95 | 5.70 |
| ABM 0%: β-1,4-mannobiose 100% | 5.90 | 7.30 |
| ABM 25%: β-1,4-mannobiose 75% | 5.70 | 5.95 |
| ABM 50%: β-1,4-mannobiose 50% | 5.75 | 5.80 |
| ABM 25%: β-1,4-mannobiose 75% | 5.75 | 6.35 |

The model studies show that mycelium of ABM on rye and β-1,4-mannobiose have a significant effect on the adhesion of Salmonella typhimurium. The onset OD600 measured for the different combinations of ABM mycelium on rye and β-1,4-mannobiose is significantly lower than the onset OD600 measured for ABM mycelium and β-1,4-mannobiose alone.

The experiments with E. coli reveal that ABM mycelium on rye considerably reduces the onset OD600 compared to the control. The experiments also show that β-1,4-mannobiose has an onset OD600 exceeding the onset observed for the control, revealing that β-1,4-mannobiose does not have an effect on the adhesion of E. coli. The combinations of ABM mycelium on rye and β-1,4-mannobiose show a considerably lower onset OD600 than the control and the β-1,4-mannobiose alone. It is further noted that the onset OD600 values of the combinations is lower than what is expected based on a linear relationship between the onset OD values of ABM mycelium on rye alone and β-1,4-mannobiose alone.

### Example 5 (reference example)

An in vivo study was conducted according to the protocol described in Example 2, except that 12 animals per treatment group were used and the piglets were selected based on the presence of cefotaxime-resistant Escherichia Coli; the selected animals were infected with Salmonella entiritidis after 5 days. In this study the shedding of cefotaxime-resistant Escherichia Coli to ABM mycelium on rye and to a combination of ABM mycelium on rye and β-1,4-mannobiose at 50:50. The organic acid blend was a regular C₁-C₁₆ organic acid blend containing a combination of formic and lactic acid. The total amount of the agents is the same in all experiments.

The results for the test with cefotaxime-resistant Escherichia Coli are shown in the Table below.

**Table 5. Effect of ABM mycelium on rye and β-1,4-mannobiose on the shedding of cefotaxime-resistant Escherichia Coli, by measuring over the first 4 and over 16 days**

| Compound | *Amount (kg*/*ton feed)* | *1-4 days* | *16 days* |
|---|---|---|---|
| Control | - | 2.0 | 2.1 |
| Acid blend | 4 | 1.4 | 1.5 |
| ABM 100% | 2 | 0.8 | 1.0 |
| ABM 50%: β-1,4-mannobiose 50% | 2 | 0.5 | 0.9 |

The study show that mycelium of ABM on rye with or without β-1,4-mannobiose have a significant effect on the shedding of cefotaxime-resistant Escherichia Coli. The acid blend alone does not provide a significant reduction of the shedding of cefotaxime-resistant Escherichia Coli.

### Example 6 (reference example)

An in vivo study was conducted using two groups, each group comprising 6 replicating pens with 30 birds. Three birds in each pen were infected with Salmonella enteritidis (seeder birds). The broilers were fed with a conventional broiler diet during 42 days. One group of broilers was treated with ABM mycelium on rye and an organic acid blend. The organic acid blend was a regular C₁-C₁₆ organic acid blend containing a combination of formic and lactic acid. The transmission of Salmonella to non-seeder birds was established by determining the number of infected or positive birds after 28 and 42 days.

After 28 days, the control (untreated) group consisted of 83% of infected birds, whereas the treated group contained 55% of infected birds. After 42 days, 60% of the birds were infected in the control group and 35% of positive birds in the treated group. This clearly demonstrates that the treatment aids in the containment of the Salmonella in the broilers.

## Claims

1. Composition comprising mycelium of Agaricus Blazei Murill (ABM), wherein the mycelium of ABM is grown on grain, and an agent affecting bacteria belonging to Enterobacteriaceae, selected from hydrolysed copra meal, yeast cell wall derived products, manno-oligosaccharides and beta-1-4-mannobiose.

2. Composition according to claim 1, wherein the agent affecting bacteria belonging to Enterobacteriaceae is β-1,4-mannobiose.

3. Composition according to any one of the preceding claims wherein the weight ratio of mycelium of ABM and the agent affecting bacteria belonging to Enterobacteriaceae in the compositions of the invention is at least 0.01 and at most 50.

4. Animal feed comprising mycelium of Agaricus Blazei Murill (ABM), wherein the mycelium of ABM is grown on grain, and an agent affecting bacteria belonging to Enterobacteriaceae, selected from hydrolysed copra meal, yeast cell wall derived products, manno-oligosaccharides and beta-1-4-mannobiose.

5. Animal feed according to claim 4, wherein the ABM mycelium is present in the animal feed in an amount between 0.01 and 5 kg per ton of total daily intake.

6. Premix of animal feed comprising mycelium of Agaricus Blazei Murill (ABM), wherein the mycelium of ABM is grown on grain, and an agent affecting bacteria belonging to Enterobacteriaceae, selected from hydrolysed copra meal, yeast cell wall derived products, manno-oligosaccharides and beta-1-4-mannobiose.

7. Feed additive comprising mycelium of Agaricus Blazei Murill (ABM), wherein the mycelium of ABM is grown on grain, and an agent affecting bacteria belonging to Enterobacteriaceae, selected from hydrolysed copra meal, yeast cell wall derived products, manno-oligosaccharides and beta-1-4-mannobiose.

8. Kit-of-parts comprising a first constituting part that comprises Agaricus Blazei Murril (ABM) mycelium, wherein the mycelium of ABM is grown on grain, and a second constituting part comprising one or more agent affecting bacteria belonging to Enterobacteriaceae, selected from hydrolysed copra meal, yeast cell wall derived products, manno-oligosaccharides and beta-1-4-mannobiose, and optionally an instruction to orally administer both these parts to an animal.

## Patentansprüche

1. Zusammensetzung, umfassend Myzel von Agaricus Blazei Murill (ABM), wobei das Myzel von ABM auf Getreide gezüchtet wird, und ein Mittel, das auf Bakterien, die zu Enterobacteriaceae gehören, einwirkt, ausgewählt aus hydrolysiertem Kopramehl, von Hefezellwänden abgeleiteten Produkten, Manna-Oligosacchariden und beta-1-4-Mannobiose.

2. Zusammensetzung nach Anspruch 1, wobei das Mittel, das auf Bakterien, die zu Enterobacteriaceae gehören, einwirkt, ß-1,4-Mannobiose ist.

3. Zusammensetzung nach einem der vorhergehenden Ansprüche, wobei das Gewichtsverhältnis von Myzel von ABM und dem Mittel, das Bakterien beeinflusst, die zu Enterobacteriaceae gehören, in den Zusammensetzungen der Erfindung mindestens 0,01 und höchstens 50 beträgt.

4. Tierfutter, umfassend Myzel von Agaricus Blazei Murill (ABM), wobei das Myzel von ABM auf Getreide gezüchtet wird, und ein Mittel, das auf Bakterien, die zu Enterobacteriaceae gehören, einwirkt, ausgewählt aus hydrolysiertem Kopra-Mehl, von Hefezellwänden abgeleiteten Produkten, Manna-Oligosacchariden und beta-1-4-Mannobiose.

5. Tierfutter nach Anspruch 4, wobei das ABM-Myzel in dem Tierfutter in einer Menge zwischen 0,01 und 5 kg pro Tonne der gesamten täglichen Aufnahme vorhanden ist.

6. Vormischung für Tierfutter, umfassend Myzel von Agaricus Blazei Murill (ABM), wobei das Myzel von ABM auf Getreide gezüchtet wird, und ein Mittel, das auf Bakterien, die zu Enterobacteriaceae gehören, einwirkt, ausgewählt aus hydrolysiertem Kopra-Mehl, von Hefezellwänden abgeleiteten Produkten, Manna-Oligosacchariden und beta-1-4-Mannobiose.

7. Futtermittelzusatz, umfassend Myzel von Agaricus Blazei Murill (ABM), wobei das Myzel von ABM auf Getreide gezüchtet wird, und ein Mittel, das auf Bakterien, die zu Enterobacteriaceae gehören, einwirkt, ausgewählt aus hydrolysiertem Kopramehl, von Hefezellwänden abgeleiteten Produkten, Manna-Oligosacchariden und beta-1-4-Mannobiose.

8. Teilesatz, umfassend einen ersten, konstituierenden Teil, der Agaricus Blazei Murril (ABM) Mycelium umfasst, wobei das Mycelium von ABM auf Getreide gezüchtet wird, und einen zweiten konstituierenden Teil, der ein oder mehrere Mittel umfasst, das/die Bakterien beeinflusst/beeinflussen, die zu Enterobacteriaceae gehören, ausgewählt aus hydrolysiertem Kopra-Mehl, von Hefezellwänden abgeleiteten Produkten, Manna-Oligosacchariden und Beta-1-4-Mannobiose, und gegebenenfalls eine Anweisung zur oralen Verabreichung dieser beiden Teile an ein Tier.

## Revendications

1. Composition comprenant du mycélium d'Agaricus Blazei Murill (ABM), dans laquelle le mycélium d'ABM est cultivé sur des grains, et un agent affectant les bactéries appartenant à la famille des Entérobactéries, sélectionné parmi la farine de coprah hydrolysée, les produits dérivés de la paroi cellulaire de levure, les manno-oligosaccharides et le bêta-1-4-mannobiose.

2. Composition selon la revendication 1, dans laquelle l'agent affectant les bactéries appartenant aux Enterobacteriaceae est le β-1,4-mannobiose.

3. Composition selon l'une quelconque des revendications précédentes, dans laquelle le rapport pondéral du mycélium d'ABM et de l'agent affectant les bactéries appartenant aux Enterobacteriaceae dans les compositions de l'invention est d'au moins 0,01 et d'au plus 50.

4. Aliments pour animaux comprenant du mycélium de Murill Agaricus Blazei (ABM), dans lesquels le mycélium d'ABM est cultivé sur des grains, et un agent affectant les bactéries appartenant à la famille des Enterobacteriaceae, sélectionné parmi la farine de coprah hydrolysée, les produits dérivés de la paroi cellulaire de levure, les manno-oligosaccharides et le bêta-1-4-mannobiose.

5. Aliments pour animaux selon la revendication 4, dans lesquels le mycélium d'ABM est présent dans les aliments pour animaux en une quantité comprise entre 0,01 et 5 kg par tonne de dose journalière totale.

6. Prémélange d'aliments pour animaux comprenant du mycélium d'Agaricus Blazei Murill (ABM), dans lequel le mycélium d'ABM est cultivé sur des grains, et un agent affectant les bactéries appartenant à la famille des Enterobacteriaceae, sélectionné parmi la farine de coprah hydrolysée, les produits dérivés de la paroi cellulaire de levure, les manno-oligosaccharides et le bêta-1-4-mannobiose.

7. Additif alimentaire comprenant du mycélium d'Agaricus Blazei Murill (ABM), dans lequel le mycélium d'ABM est cultivé sur des grains, et un agent affectant les bactéries appartenant à la famille des Enterobacteriaceae, sélectionné parmi la farine de coprah hydrolysée, les produits dérivés de la paroi cellulaire de levure, les manno-oligosaccharides et le bêta-1-4-mannobiose.

8. Kit de pièces comprenant une première partie constitutive qui comprend le mycélium d'Agaricus Blazei Murril (ABM), dans lequel le mycélium d'ABM est cultivé sur des grains, et une seconde partie constitutive comprenant un ou plusieurs agents affectant les bactéries appartenant à la famille des Entérobactéries, sélectionnées parmi la farine de coprah hydrolysée, les produits dérivés de la paroi cellulaire de levure, les manno-oligosaccharides et le bêta-1-4-mannobiose, et éventuellement une instruction d'administrer oralement ces deux parties à un animal.
